(19) **Europäisches Patentamt**

**European Patent Office**

**Office européen des brevets**

(11) Veröffentlichungsnummer: **0 183 674**
**B1**

(12) **EUROPÄISCHE PATENTSCHRIFT**

(45) Veröffentlichungstag der Patentschrift:
**10.05.89**

(21) Anmeldenummer: **85890227.3**

(22) Anmeldetag: **19.09.85**

(51) Int. Cl.⁴: **A 61 L 2/04 //**
**A61L25/00, A61K35/16**

(54) Verfahren zur Inaktivierung von vermehrungsfähigen filtrierbaren Krankheitserregern.

(30) Priorität: **28.09.84 AT 3084/84**

(43) Veröffentlichungstag der Anmeldung:
**04.06.86 Patentblatt 86/23**

(45) Bekanntmachung des Hinweises auf die Patenterteilung:
**10.05.89 Patentblatt 89/19**

(84) Benannte Vertragsstaaten:
**AT BE CH DE FR GB IT LI NL SE**

(56) Entgegenhaltungen:
**EP-A-0 018 561**
**EP-A-0 068 149**
**EP-A-0 094 611**
**EP-A-0 142 059**
**WO-A-82/03871**
**GB-A-2 041 942**

(73) Patentinhaber: **IMMUNO Aktiengesellschaft für chemisch- medizinische Produkte,
Industriestrasse 72, A-1220 Wien (AT)**

㉒. Erfinder: **Seelich, Thomas, Dr., Gerhardusgasse 10/10, A-1200 Wien (AT)**

(74) Vertreter: **Wolfram, Gustav, Dipl.- Ing.,
Schwindgasse 7 P.O. Box 205, A-1041 Wien (AT)**

## Beschreibung

Die Erfindung betrifft ein Verfahren zur Inaktivierung von vermehrungsfähigen filtrierbaren Krankheitserregern in Fibrinogen und Faktor XIII enthaltenden Gewebeklebstoffen bei weitgehender Erhaltung ihrer biologischen Aktivität.

Gewebeklebstoffe werden aus menschlichem oder tierischem Blut bzw. Plasma hergestellt und dienen zur physiologischen Verklebung von Gewebe- oder Knochenteilen, Wundversiegelung, Blutstillung und Förderung der Wundheilung. Sie enthalten als wirksame Bestandteile Fibrinogen und Faktor XIII und können gegebenenfalls weitere Plasmaproteine, wie z. B. Fibronectin, Albumin, Plasminogen-Aktivator-Inhibitoren und/oder Plasmin-Inhibitoren, sowie weitere Zusätze enthalten. Ein derartiger Gewebeklebstoff ist z. B. in der GB-A-2 041 942 beschrieben.

Die Wirkungsweise dieser Gewebeklebstoffe beruht auf der Umwandlung von löslichem Fibrinogen in unlösliches Fibrin, auf der Aktivierung von Faktor XIII zu Faktor XIIIa durch Thrombin in Gegenwart von $Ca^{2+}$-Ionen und der Vernetzung des gebildeten Fibrins durch Faktor XIIIa zu einem Hochpolymer. Der Vernetzung des Fibrins, insbesondere der Fibrin-α-Ketten, kommt dabei besondere Bedeutung zu, weil dadurch die Festigkeit des gebildeten Clots und damit der Klebung wesentlich erhöht wird (L. L.Shen, R. P. McDonagh, J. Hermans jr.: "Fibringel structure influence of calcium and covalent crosslinking on the elasticity". Biochem. Biophys. Res. Comm. 56, 793 - 798, 1974; T. Seelich, H. Redl: "Theoretische Grundlagen des Fibrinklebers" in: K. Schimpf: "Fibrinogen, Fibrin und Fibrinkleber", F. K. Schattauer Verlag, Stuttgart-New York, 199 - 208, 1980).

Die Qualität eines Gewebeklebstoffes hängt somit wesentlich von dessen Gehalt an löslichem, intaktem, mit Thrombin gerinnbarem Fibrinogen und von der Vernetzbarkeit des gebildeten Fibrins durch den vorhandenen Faktor XIII ab.

Trotz sorgfältiger überprüfung der Ausgangsprodukte bei der Herstellung von Blutpräparationen ist das Risiko, daß Patienten nach einer Behandlung an mikrobiellen Infektionen erkranken, nicht gering. Es gibt zwar mehrfach Bemühungen, Krankheitserreger zu inaktivieren, doch ist die Sicherheit der Präparation nicht immer gewährleistet bzw. wird die biologische Aktivität der Wirkstoffe beeinträchtigt.

Bei Präparationen, die für Infusionszwecke bestimmt sind, ist es möglich, Verluste an biologischer Aktivität durch entsprechend höhere Dosierung zu kompensieren, was zwar die Wirtschaftlichkeit, nicht aber die Wirksamkeit der Produkte beeinträchtigt; bei Gewebeklebstoffen hingegen ist es nicht möglich, eine verminderte biologische Aktivität (ausgedrückt im Gehalt an intaktem, löslichem, mit Thrombin gerinnbarem Fibrinogen und der Vernetzbarkeit des daraus gebildeten Fibrins) durch höhere Dosierung zu kompensieren; denn eine Klebung wird keinesfalls dadurch fester, daß man mehr Klebstoff von minderer Qualität einsetzt. Darüber hinaus ist bekannt, daß vernetztes Fibrin das Fibroblastenwachstum und damit die Wundheilung stimuliert, während Fibrinogen oder unvernetztes Fibrin diese erwünschte Wirkung kaum zeigen (S. Kasai, T. Kunimoto, K. Nitta: "Cross-linking of fibrin by activated factor XIII stimulates attachment, morphological changes and proliferation of fibroblasts", Biomed Res 4, 155 - 160, 1983).

Aus der vorstehenden Darstellung ergibt sich die Forderung, daß Inaktivierungsverfahren für Gewebeklebstoffpräparationen nicht nur eine hohe Wirksamkeit gegenüber vermehrungsfähigen filtrierbaren Krankheitserregern, wie Viren, aufweisen müssen, sondern auch, daß die biologische Aktivität der Präparationen weitgehend erhalten bleibt.

Es sind verschiedene Verfahren bekannt, bei welchen Blutprodukte zwecks Inaktivierung von vermehrungsfähigen filtrierbaren Krankheitserregern in Blutprodukten einer Hitzebehandlung unterworfen werden.

So ist in der veröffentlichten PCT-Anmeldung WO 82/03871 ein Verfahren zur Behandlung von Blutgerinnungsenzyme enthaltenden Zubereitungen beschrieben, wobei diese zur Inaktivierung vorhandener infektiöser Viren im trockenen Zustand erhitzt werden; unter "trockenem Zustand" wird ein Wassergehalt bis zu 0,05 (5 Gew.-%) verstanden. Die dort angeführten Zubereitungen enthalten jedoch kein Fibrinogen und keinen Faktor XIII; es handelt sich um keine Gewebeklebstoff-Präparationen.

Ein ähnliches Verfahren ist in der EP-A-0 094 611 für Faktor VIII-hältige Kompositionen beschrieben.

Rosenberg et al. beschreiben in einer Abhandlung des XII. International Congress on Blood Transfusion, Abstracts, "MIR" Publishers, Moscow 1969, pp. 473 - 475 ein Verfahren zur Inaktivierung von albuminhältigen Präparationen und Fibrinogen in trockenem Zustand durch 10-stündiges Erhitzen auf 60°C. Diese intravenös zu verabreichenden Präparationen waren zur Behandlung von Fibrinogen-Mangelzuständen bestimmt, wobei das Fibrinogen löslich und gerinnbar sein mußte. Diese Präparationen enthielten keinen Faktor XIII und waren nicht zur Gewebeklebung bestimmt.

Des weiteren ist gemäß der EP-A-103 196 ein Verfahren zur Herstellung einer Gewebeklebstoffpräparation bekannt, bei welchem während des Fraktionierungsprozesses eine Gewebeklebstofflösung in Gegenwart von Calciumionen sowie großen Mengen an Saccharose und Glycin als Stabilisatoren erhitzt wird; das Verfahren hat allerdings den Nachteil, daß während der Erhitzung große Mengen an Stabilisierungsmitteln zugesetzt und danach wieder entfernt werden müssen. Dabei wird ein Teil des Fibrinogens denaturiert und muß nach dem Erhitzungsschritt abgetrennt werden. Darüber hinaus haben Behandlungen dieser Art den Nachteil, daß die zugesetzten Stabilisatoren nicht nur die Proteine, sondern auch die eventuell vorhandenen Viren gegenüber Hitze stabilisieren. Die Wirksamkeit solcher Verfahren ist daher keinesfalls mit der Wirksamkeit der standardisierten Hitzebehandlung von Albuminlösungen (10-stündiges Erhitzen auf 60°C) zu vergleichen, da diese wegen der außergewöhnlich hohen Hitzestabilität des Albuminmoleküls ohne Zusatz größerer Mengen

2

an Stabilisatoren durchgeführt werden kann.

In der EP-A-0 142 059 ist ein Verfahren zur Hitzebehandlung von viruskontaminierten Plasmaproteinen, darunter die Gerinnungsfaktoren II, V, VII, VIII, IX, X bzw. Fibrinogen, Plasminogen und Thrombin beschrieben, wobei die Präparationen im trockenen Zustand in Gegenwart einer sauren Aminosäure und einer basischen Aminosäure erhitzt werden. Von der Kombination der Säuren wird eine stabilisierende Wirkung auf die Plasmaproteine erwartet; diese stabilisierende Wirkung erstreckt sich jedoch auch auf die Viren, so daß die Inaktivierung verzögert bzw. erschwert wird.

Die Erfindung bezweckt die Vermeidung der Nachteile der bekannten Inaktivierungsverfahren und stellt sich die Aufgabe, eine Gewebeklebstoffpräparation menschlichen oder tierischen Ursprungs zu schaffen, die eine hohe Sicherheit gegenüber vermehrungsfähigen filtrierbaren Krankheitserregern, wie Viren, aufweist und deren biologische Aktivität weitgehend erhalten bleibt, d. h. daß das Fibrinogen in hoher Konzentration löslich und mit Thrombin gerinnbar bleibt und das gebildete Fibrin eine hohe Vernetzbarkeit aufweist. Im besonderen soll nach einer Behandlung mit dem erfindungsgemäß hergestellten Gewebeklebstoff nach der Wundbehandlung eine ausreichende Festigkeit der Klebestelle und eine rasche, komplikationslose Wundheilung gewährleistet sein.

Zur Lösung dieser Aufgabe besteht das erfindungsgemäße Verfahren darin, daß Gewebeklebstoffpräparationen, die einen Mindestgehalt von 100 Einheiten Faktor XIII/g Fibrinogen aufweisen, in Gegenwart eines sauerstofffreien inerten Schutzgases, vorzugsweise Stickstoff, oder unter Vakuum im trockenen Zustand bei einer Temperatur im Bereich von 50 bis 120°C während einer Dauer von mindestens 1 min erhitzt werden. Unter "trockenem Zustand" wird ein Wassergehalt bis zu 0,05 (5 Gew.-%) verstanden. Dabei entspricht der höchsten Temperatur die kürzeste Erhitzungszeit und umgekehrt. Die Hitzebehandlung kann in irgendeiner Stufe des Herstellungsprozesses und/oder nach Abfüllung der Präparation in Endbehälter durchgeführt werden.

Der "trockene Zustand" der Gewebeklebstoffpräparation kann durch Nachtrocknen mit wasserbindenden Mitteln, wie Phosphorpentoxid, herbeigeführt bzw. sichergestellt werden.

Nach einer bevorzugten Ausführungsform wird eine Gewebeklebstoffpräparation durch Fraktionieren von humanem Kryopräzipitat hergestellt und zusätzlich zu dem nativen Gehalt an Faktor XIII weiterer Faktor XIII, vorzugsweise bis zum Erreichen eines Gesamtgehaltes an Faktor XIII von etwa 500 Einheiten/g Fibrinogen, zugegeben, wobei die Hitzebehandlung in irgendeiner Stufe des Fraktionierungsprozesses und/oder nach Abfüllung der Präparation in Endbehälter durchgeführt wird.

Die hitzebehandelte Präparation kann vorteilhaft in trockenem Zustand in Endbehältern gelagert und vor der Anwendung zu einer Konzentration von mindestens 70 mg Fibrinogen pro ml gelöst werden.

Es ist aber auch möglich, das Produkt nach dem Erhitzungsschritt zu lösen, in Endbehälter abzufüllen und zur Haltbarmachung in tiefgefrorenem Zustand zu lagern.

Das erfindungsgemäße Verfahren beruht auf neuen Erkenntnissen, uzw.:

1) Die Stabilität (thermische Belastbarkeit) der Präparationen während der Hitzebehandlung unter einem sauerstofffreien Schutzgas oder unter Vakuum ist wesentlich höher als bei Luftzutritt.

2) Die Stabilität (thermische Belastbarkeit) von Gewebeklebstoffpräparationen nimmt mit steigendem Faktor XIII-Gehalt zu, mit steigendem Wassergehalt (Restfeuchte) hingegen ab.

3) Der Inaktivierungsgrad der Hitzebehandlung in trockenem Zustand in sauerstofffreier Atmosphäre ist im wesentlichen gleich hoch wie unter Luftzutritt.

Diese Tatsachen werden durch die Ergebnisse der nachfolgenden Versuche erläutert;

Versuch 1:
Stabilität von Gewebeklebstoffpräparationen bei unterschiedlichen Wassergehalten einerseits unter Stickstoff oder unter Vakuum, andererseits unter Luft

Eine Gewebeklebstoffpräparation wurde in bekannter Weise nach dem Verfahren gemäß der AT-B-359 652 bereitet: 277 l humanes, bei -20° eingefrorenes Frischplasma wurden auf +2°C erwärmt, das entstandene Kryopräzipitat durch Zentrifugieren abgetrennt und mit einer Pufferlösung vom pH-Wert 6,5, enthaltend 6,6 g Na$_3$-Citrat.2H$_2$O, 3,4 g NaCl, 10,0 g Glycin, 25.000 KIE Aprotinin und 200 I.E. Heparin pro l, behandelt und nochmals bei +2°C zentrifugiert. Der abgetrennte Niederschlag wurde in einer weiteren Pufferlösung vom pH-Wert 7,9, enthaltend 19,0 g Humanalbumin, 9,0 g Glycin, 1,0 g Trinatriumcitrat.2H$_2$O, 25.000 KIE Aprotinin und 200 I.E. Heparin pro l, gelöst und auf eine Proteinkonzentration von 50 g pro Liter eingestellt. Diese Lösung wurde sterilfiltriert, in Endbehälter (Glasfläschchen) zu je 12,5 ml abgefüllt, tiefgefroren und gefriergetrocknet. In der so erhaltenen Präparation betrug das Verhältnis Faktor XIII: Fibrinogen, ausgedrückt in Einheiten Faktor XIII pro Gramm Fibrinogen, 109; der Wassergehalt (Restfeuchte) der Präparation betrug weniger als 0,01 (1 Gew.-%).

Der Gehalt an Fibrinogen wurde entsprechend der Vorschrift nach USP XVI, Seite 298, als das durch Thrombin gerinnbare Protein nach der Methode Kjeldahl bestimmt.

Der Gehalt an Faktor XIII wurde mittels eines Fibrin-Vernetzungstests, bei dem Faktor XIII-freies Fibrinogen als Substrat verwendet wird, auf folgende Weise bestimmt:

Je 0,5 ml einer Faktor XIII-freien Fibrinogenlösung mit einem Fibrinogengehalt von 10 mg/ml werden mit je 0,05 ml von verschiedenen Verdünnungen der zu bestimmenden Probe und je 0,1 ml einer Lösung, enthaltend 60 I. E. Thrombin und 130 µMol $CaCl_2$ pro ml, vermischt und bei 37°C inkubiert. Nach einer Inkubationszeit von 2 Stunden wird die Reaktion durch Zugabe eines Gemisches von Harnstoff, Na-Dodecylsulfat (SDS) und β-Mercaptoäthanol gestoppt und werden die in den Proteinen enthaltenen Disulfidbrücken reduktiv gespalten. Der Vernetzungsgrad der Fibrin-γ-Ketten wird nach SDS-Polyacrylamid-Gelelektrophorese (K. Weber, M. Osborn: "The reliability of molecular weight determinations by dodecyl sulfate-polyacrylamide-gel electrophoresis", J. Biol. Chem. <u>244</u>, 4406 - 4412, 1969) der so erhaltenen Proben und Färbung mit Coomassie-Blau densitometrisch bestimmt und dient als Maß für den Faktor XIII-Gehalt.

Als Standard dient gepooltes humanes Citratplasma, wobei per definitionem 1 ml Plasma 1 Einheit Faktor XIII enthält. Diejenigen Verdünnungen der Probe und des Standards, die unter den Testbedingungen eine 50 %-ige Vernetzung der Fibrin-γ-Ketten bewirken, werden bestimmt und der Faktor XIII-Gehalt der ursprünglichen Probe nach der Formel

$$X = \frac{V_x}{V_s}$$

berechnet, wobei

$V_x$ die Verdünnung der unbekannten Probe und

$V_s$ die Verdünnung des Standards ist.

Der Wassergehalt wurde durch Extraktion mit wasserfreiem Methanol bestimmt, uzw. nach der Methode nach Karl Fischer in einem Titrator nach dem coulometrischen Verfahren (E. Scholz, Fresenius' Z. anal. Chem. <u>314</u>, 567 - 571, 1983).

Ein Teil der in beschriebener Weise erhaltenen gefriergetrockneten Gewebeklebstoffpräparation wurde zu einer Proteinkonzentration von 50 mg/ml gelöst, in Glasfläschchen zu je 2,5 ml abgefüllt, tiefgefroren und neuerlich gefriergetrocknet.

Anschließend wurden Proben mit verschiedenem Wassergehalt eingestellt, uzw. mit 0,007 (0,7 Gew.-%), 0,03 (3 Gew.-%) bzw. 0,05 (5 Gew.-%). Die Proben mit einem Wassergehalt von 0,007 wurden entweder unter Vakuum (<10 Pa, d. s. (<0,1 mbar) oder unter $N_2$ oder unter Luft bei Normaldruck luftdicht verschlossen. Die Proben mit einem Wassergehalt von 0,03 und 0,05 wurden entweder unter $N_2$ oder unter Luft verschlossen.

Mehrere Proben jeder Art wurden sodann 10 Stunden lang auf 60°C erhitzt und sodann die Restaktivität, d. i. der Gehalt an intaktem, mit Thrombin gerinnbarem Fibrinogen und die Vernetzbarkeit der Fibrin-α-Ketten als Maß für die biologische Aktivität bestimmt, wobei die gefriergetrockneten, erhitzten bzw. nicht erhitzten Proben mit je 1,0 ml destilliertem Wasser gelöst wurden.

Die Bestimmung der Vernetzbarkeit der Fibrin-α-Ketten erfolgte nach einem Vernetzungstest (T. Seelich, H. Redl: "Theoretische Grundlagen des Fibrinklebers" in K. Schimpf: "Fibrinogen, Fibrin und Fibrinkleber", F. K. Schattauer Verlag, Stuttgart-New York, 199 - 208, 1980), bei dem nach Vermischen des gelösten, gebrauchsfertigen Gewebeklebstoffes mit dem gleichen Volumen einer Lösung, enthaltend 40µ Mol $CaCl_2$ und 15 I. E. Thrombin pro ml, die Mischung bei 37°C inkubiert wird. Der Vernetzungsgrad der Fibrin-α-Ketten wird nach Stoppen der Reaktion und reduktiver Spaltung der in den Proteinen enthaltenen Disulfid-Brücken durch Zugabe eines Gemisches von Harnstoff, Na-Dodecylsulfat (SDS) und β-Mercaptoäthanol mittels SDS-Polyacrylamid-Gelelektrophorese (K. Weber, M. Osborn: "The reliability of molecular weight determinations by dodecyl sulfatepolyacrylamide gel electrophoresis", J. Biol. Chem. <u>244</u>, 4406 - 4412, 1969) und Färbung mit Goomassie-Blau densitometrisch bestimmt.

Die erhaltenen Ergebnisse sind in der Tabelle 1 zusammengestellt.

Eine nicht erhitzte Probe diente als Vergleich für die unter unterschiedlichen Bedingungen erhitzten Proben.

Ein Vergleich der gefundenen Werte für die Vernetzung der Fibrin-α-Ketten zeigt deutlich, daß erstens die Stabilität des Gewebeklebstoffes beim Erhitzen unter $N_2$ oder unter Vakuum wesentlich besser ist als unter Luft und zweitens die Stabilität mit steigendem Wassergehalt abnimmt.

<u>Tabelle 1</u>

Stabilität eines Gewebeklebstoffes mit unterschiedlichen Wassergehalten beim Erhitzen auf 60°C unter $N_2$, Vakuum oder Luft.

| Wasserge-gehalt | Erhitzung auf 60°C | | Restaktivität | |
| | Stunden | Atmosphäre | Fibrinogen (Gerinnbares Protein) (mg/ml) | Vernetzung der Fibrin-α-Ketten nach 2 Stunden |
|---|---|---|---|---|
| 0,007 | 0 | $N_2$ | 85 | 0,80 |
| 0,007 | 10 | $N_2$ | 84 | 0,76 |
| 0,007 | 10 | Vakuum | 84 | 0,74 |
| 0,007 | 10 | Luft | 80 | 0,59 |
| 0,03 | 10 | $N_2$ | 83 | 0,60 |
| 0,03 | 10 | Luft | 80 | 0,21 |
| 0,05 | 10 | $N_2$ | 84 | 0,24 |
| 0,05 | 10 | Luft | 79 | 0,11 |

**Versuch 2:**

Stabilität von Gewebeklebstoffpräparationen mit unterschiedlichem Gehalt an Faktor XIII, ausgedrückt durch das Verhältnis Einheiten Faktor XIII pro Gramm Fibrinogen:

Eine Gewebeklebstoffpräparation wurde in gleicher Weise hergestellt wie bei Versuch 1. Ein Teil davon wurde zu einer Proteinkonzentration von 50 mg/ml aufgelöst; die Lösung wurde in zwei Teile geteilt.

Ein Teil wurde mit 15 Einheiten Faktor XIII pro ml versetzt. Beide Teile wurden sodann zu je 2,5 ml in eine Anzahl von Glasfläschchen abgefüllt, tiefgefroren, gefriergetrocknet und unter $N_2$ verschlossen. Der Gehalt an Fibrinogen und Faktor XIII wurde, wie in Versuch 1 beschrieben, bestimmt und der Gehalt an Faktor XIII pro Gramm Fibrinogen für jede der beiden Präparationen berechnet.

Mehrere Proben der beiden Präparationen wurden bei 60°C bzw. bei 80°C verschieden lang erhitzt. Je zwei Proben wurden vor dem Erhitzen bzw. zu bestimmten Zeiten während des Erhitzungsvorganges zur Messung der Restaktivität entnommen.

Die Bestimmung der Restaktivität erfolgte, wie in Versuch 1 beschrieben. Die Ergebnisse sind in der Tabelle 2 zusammengestellt.

Ein Vergleich der gefundenen Werte für die Vernetzung der Fibrin-$\alpha$-Ketten zeigt deutlich, daß die Stabilität von Gewebeklebstoffpräparationen mit steigendem Gehalt an Faktor XIII, ausgedrückt als Verhältnis Einheiten Faktor XIII Gramm Fibrinogen, zunimmt.

## Tabelle 2

Stabilität von Gewebeklebstoffpräparationen in Abhängigkeit vom Verhältnis Faktor XIII : Fibrinogen beim Erhitzen

| Faktor XIII: Fibrinogen (E/g) | Erhitzung Stunden | °C | Fibrinogen (Gerinnbares Protein) (mg/ml) | Restaktivität Vernetzung der Fibrin-$\alpha$-Ketten nach 2 Stunden |
|---|---|---|---|---|
| 109 | 0 | - | 85 | 0,81 |
| 109 | 30 | 60 | 83 | 0,57 |
| 109 | 100 | 60 | 84 | 0,50 |
| 109 | 10 | 80 | 85 | 0,67 |
| 109 | 30 | 80 | 84 | 0,48 |
| 489 | 0 | - | 83 | 0,95 |
| 489 | 30 | 60 | 84 | 0,87 |
| 489 | 100 | 60 | 83 | 0,85 |
| 489 | 10 | 80 | 82 | 0,79 |
| 489 | 30 | 80 | 84 | 0,75 |
| 489 | 100 | 80 | 84 | 0,69 |

**Versuch 3:**

Stabilität von Gewebeklebstoffpräparationen mit einem Faktor XIII-Gehalt von etwa 500 Einheiten pro Gramm Fibrinogen und einem Wassergehalt von 0,005 beim Erhitzen unter $N_2$ auf Temperaturen bis zu 120°C:

Die Herstellung der Gewebeklebstoffpräparation erfolgte in gleicher Weise wie bei den Versuchen 1 und 2. Der Wassergehalt der Präparation wurde mit 0,005 und das Verhältnis Einheiten Faktor XIII : Gramm Fibrinogen Mit 496 bestimmt. Proben der Präparation wurden bei verschiedenen Temperaturen im Bereich zwischen 60 C und 120°C verschieden lang erhitzt. Je zwei Proben wurden vor dem Erhitzen bzw. nach bestimmten Erhitzungszeiten zur Messung der Restaktivität entnommen. Die Bestimmung der Restaktivität erfolgte, wie bei Versuch 1 beschrieben.

Die Ergebnisse sind in der Tabelle 3 zusammengestellt.

Unter Zugrundelegung der Qualitätsmerkmale, die für Gewebeklebstoffe auf Basis von Fibrinogen und Faktor XIII zu fordern sind, nämlich einem Mindestgehalt an Fibrinogen von 70 mg/ml und eine Vernetzung der Fibrin-$\alpha$-Ketten in dem beschriebenen Vernetzungstest von mindestens 0,35 (35 %), ergibt sich somit, daß solche Gewebeklebstoffpräparationen unter den angeführten Bedingungen beträchtlich länger als in diesem Versuch erhitzt werden können, ohne zu große Qualitätsverluste in Kauf nehmen zu müssen.

Tabelle 3

Stabilität einer Gewebeklebstoffpräparation mit einem Faktor XIII-Gehalt von 496 Einheiten pro Gramm Fibrinogen und einem Wassergehalt von 0,005 beim Erhitzen unter $N_2$.

| Erhitzung Stunden °C | | Restaktivität | |
|---|---|---|---|
| | | Fibrinogen (Gerinnbares Protein) (mg/ml) | Fibrin-$\alpha$-Vernetzung nach 2 Stunden |
| 0 | - | 86 | 0,95 |
| 10 | 60 | 83 | 0,90 |
| 30 | 60 | 84 | 0,87 |
| 100 | 60 | 84 | 0,83 |
| 10 | 80 | 85 | 0,80 |
| 30 | 80 | 85 | 0,77 |
| 100 | 80 | 83 | 0,70 |
| 3 | 90 | 84 | 0,90 |
| 1 | 100 | 83 | 0,81 |
| 0,3 | 110 | 84 | 0,85 |
| 0,1 | 120 | 84 | 0,81 |

Das erfindungsgemäße Verfahren einschließlich der Inaktivierung von vermehrungsfähigen Krankheitserregern ist in den folgenden Beispielen näher erläutert. Da die notwendigen Untersuchungen über Virusinaktivierungen in Blutprodukten nicht sofort am Menschen vorgenommen werden können, erfolgt die Bewertung der Wirksamkeit der Inaktivierung mit Hilfe von Modellviren.

**Beispiel 1:**

Inaktivierung eines Modellvirus (Sindbis-Virus) und Restaktivität einer Gewebeklebstoffpräparation beim trockenen Erhitzen unter $N_2$ oder Vakuum einerseits und unter Luft andererseits:

Eine Gewebeklebstoffpräparation wurde in gleicher Weise, wie bei Versuch 1 beschrieben, hergestellt.

Ein Teil der erhaltenen gefriergetrockneten Gewebeklebstoffpräparation wurde zu einer Proteinkonzentration von 50 mg/ml gelöst, mit einer Sindbis-Virussuspension in Zellkulturmedium TCM 199 bzw. mit virusfreiem Zellkulturmedium versetzt, in Glasfläschchen zu je 2,6 ml abgefüllt, tiefgefroren und bis zu einem Wassergehalt von 0,005 (0,5 Gew.-%) gefriergetrocknet. Ein Teil der Proben wurde unter Vakuum ($<$ 10 Pa), ein weiterer Teil unter $N_2$ und ein weiterer Teil unter Luft bei Normaldruck dicht verschlossen.

Die verschlossenen Proben wurden bei einer Temperatur von 60°C verschieden lang erhitzt. Je drei Proben wurden vor dem Erhitzen und zu bestimmten Zeiten während des Erhitzungsvorganges zur Messung des Virustiters einerseits und der Restaktivität andererseits entnommen.

Die Bestimmung des Virustiters wurde in folgender Weise durchgeführt:

Die Proben wurden in je 1,0 ml Wasser gelöst und mit isotoner Kochsalzlösung im Verhältnis 1 : 10 seriell verdünnt. Der Titer des Sindbis-Virus wurde durch Bewertung des zytopathischen Effektes auf sensitive Vero-Zellen in der Mikrotiterplatte bestimmt. Die Ergebnisse wurden nach statistischer Behandlung der Auswertung entsprechend der Formel von Reed und Muench als Logarithmus $TCID_{50}$ ausgedrückt (J. L. Reed, H. Muench; Amer. J. Hyg. 27, 493, 1938).

Die Bestimmung der Restaktivität erfolgte, wie im Versuch 1 beschrieben. Die Ergebnisse sind in der Tabelle 4 zusammengestellt. Sie zeigen nicht nur, daß es möglich ist, Sindbis-Virus in Gewebeklebstoffpräparationen der beschriebenen Art vollständig zu inaktivieren, sondern führen vor allem auch zu der wichtigen Erkenntnis, daß das Verhältnis zwischen Virusinaktivierung einerseits und Restaktivität des Präparates andererseits dadurch wesentlich verbessert werden kann, daß die Erhitzung unter Ausschluß von Sauerstoff, d. h. unter einem inerten Schutzgas oder unter Vakuum durchgeführt wird.

## Tabelle 4

Inaktivierung eines Modellvirus (Sindbis-Virus) in einer Gewbeklebstoffpräparation durch trockenes Erhitzen unter $N_2$, Vakuum oder Luft

| | | | Restaktivität | | |
|---|---|---|---|---|---|
| Erhitzung auf 60°C Stunden Atmosphäre | Sindbis-Virustiter $log_{10}$ TCID$_{50}$) | Fibrinogen (Gerinnbares Protein) (mg/ml) | Vernetzung der nach 2 Stunden | Fibrin-$\alpha$-Ketten | |
| 0 | $N_2$ | 4,9 | 83 | 0,78 | |
| 3 | $N_2$ | 4,2 | 85 | 0,65 | |
| 10 | $N_2$ | 3,0 | 82 | 0,62 | |
| 30 | $N_2$ | <1 | 84 | 0,58 | |
| 0 | Vakuum | 5,0 | 83 | 0,78 | |
| 3 | Vakuum | 4,2 | 83 | 0,67 | |
| 10 | Vakuum | 2,9 | 84 | 0,60 | |
| 30 | Vakuum | <1 | 85 | 0,57 | |
| 0 | Luft | 5,0 | 83 | 0,78 | |
| 3 | Luft | 4,0 | 82 | 0,60 | |
| 10 | Luft | 2,9 | 82 | 0,51 | |
| 30 | Luft | 1,5 | 81 | 0,39 | |

**Beispiel 2:**

Die Arbeitsweise nach Beispiel 1 wurde wiederholt, mit dem Unterschied, daß die Proben nach erfolgter Gefriertrocknung in Gegenwart eines $P_2O_5$-haltigen Trocknungsmittels unter Vakuum bis zu einem Wassergehalt von 0,002 nachgetrocknet, unter Vakuum (<10 Pa) verschlossen und verschieden lang auf 70°C erhitzt wurden.

Die erhaltenen Ergebnisse sind in Tabelle 5 zusammengefaßt.

## Tabelle 5

Inaktivierung eines Modellvirus (Sindbis-Virus) in einer Gewebeklebstoffpräparation mit einem Wassergehalt von 0,002 beim Erhitzen unter Vakuum auf 70°C

| | | Restaktivität | | |
|---|---|---|---|---|
| Erhitzung Stunden 70°C | Sindbis-Virus-Titer (log$_{10}$ TCID$_{50}$) | Fibrinogen (Gerinnbares Protein) (mg/ml) | Vernetzung der nach 2 Stunden | Fibrin-$\alpha$-Ketten |
| 0 | 4,0 | 81 | 0,79 | |
| 1 | 3,1 | 80 | 0,74 | |
| 3 | 1,6 | 81 | 0,67 | |
| 10 | <1 | 82 | 0,55 | |

**Beispiel 3:**

Inaktivierung von drei verschiedenen Modellviren in einer Gewebeklebstoffpräparation durch trockenes Erhitzen auf 60°C unter $N_2$

Eine Gewebeklebstoffpräparation wurde, wie in Versuch 1 beschrieben, hergestellt und der verdünnten Lösung des Gewebeklebstoffes vor der Sterilfiltration 12 Einheiten Faktor XIII pro ml zugegeben. Nach der Gefriertrocknung enthielt die Präparation 520 Einheiten Faktor XIII pro Gramm Fibrinogen.

Ein Teil der erhaltenen Präparation wurde zu einer Konzentration von 50 mg Protein pro ml gelöst und je ein Teil der Lösung mit einer Suspension von Sindbis-Virus bzw. einer Suspension von VSV (vesicular stomatitis virus) bzw. einer Suspension von LCM (lymphotropes choriomeningitis)-Virus in Zellkulturmedium TCM 199 oder mit virusfreiem Zellkulturmedium versetzt, in Glasfläschchen zu je 2,6 ml abgefüllt, tiefgefroren, bis zu einem Wassergehalt von 0,006 gefriergetrocknet und unter $N_2$ verschlossen. Proben der Präparationen wurden auf 60°C erhitzt und wie in Beispiel 1 beschrieben vor der Erhitzung und nach bestimmten Erhitzungszeiten der Virustiter und die Restaktivität bestimmt.

Die Ergebnisse sind in Tabelle 6 zusammengefaßt.

Das Ergebnis von Beispiel 1, nämlich, daß Sindbis-Virus in einer Gewebeklebstoffpräparation durch 30-stündiges trockenes Erhitzen auf 60°C vollständig inaktiviert werden kann, wurde nach diesem Beispiel nochmals bestätigt. LCM-Virus und VSV waren bereits nach 10-stündigem Erhitzen vollständig inaktiviert.

Die Aktivität der Gewebeklebstoffpräparation war nach 30-stündigem Erhitzen noch fast vollständig erhalten.

## Tabelle 6

Inaktivierung von drei verschiedenen Modellviren in einer Gewebeklebstoffpräparation mit einem Faktor XII-Gehalt von 520 Einheiten pro Gramm Fibrinogen und einem Wassergehalt von 0,006 beim Erhitzen unter $N_2$ auf 60°C

| Erhitzung Stunden 60°C | Virus-Titer ($\log_{10}$ TCID$_{50}$) | | | Restaktivität | | |
| | | | | Fibrinogen (Gerinnbares Protein) (mg/ml) | Vernetzung der nach 2 Stunden | Fibrin-α-Ketten |
| | Sindbis | LCM | VSV | | | |
| 0 | 5,7 | 3,0 | 3,2 | 83 | 0,93 | |
| 1 | | | 2,7 | | | |
| 3 | 4,4 | 1,6 | 1,7 | | | |
| 10 | 2,4 | <1 | <1 | | | |
| 30 | <1 | <1 | <1 | 84 | 0,85 | |

## Beispiel 4:

Inaktivierung des Bakteriophagen X 174 beim trockenen Erhitzen einer Gewebeklebstoffpräparation auf 90°C

Eine Gewebeklebstoffpräparation wurde, wie in Beispiel 3 beschrieben, hergestellt.

Ein Teil der erhaltenen Präparation wurde zu einer Proteinkonzentration von 50 mg/ml gelöst und die Lösung mit einer Suspension des Bakteriophagen X 174 in Kulturmedium für Escherichia coli 11303 bzw. mit virusfreiem Kulturmedium versetzt, in Glasfläschchen zu je 2,6 ml abgefüllt, tiefgefroren, bis zu einem Wassergehalt von 0,005 gefriergetrocknet und unter $N_2$ verschlossen.

Ein Teil der Proben wurde verschieden lang auf 90°C erhitzt und der Virustiter und die Restaktivität vor dem Erhitzen und zu bestimmten Zeiten während des Erhitzungsvorganges bestimmt.

Die Bestimmung des Virustiters wurde in folgender Weise durchgeführt:

Die Proben wurden in je 1,0 ml $H_2O$ gelöst und mit 1 mM $MgCl_2$ im Verhältnis 1 : 10 seriell verdünnt. Eine Mischung von 3 ml 0,7 % Bacto-Agar, 43 bis 45°C, 100 µl Suspension von E. coli in Flüssigmedium und 200 µl der bakteriophagenhältigen Probe bzw. Verdünnung wurden durchgemischt und rasch auf einer Nähragarplatte (ATCC 129) ausgegossen. Nach Inkubation über Nacht bei 37°C wurden mit Hilfe eines Zählgerätes die durch vermehrungsfähige Viruspartikel erzeugten Plaques in konfluenten Zellrasen gezählt (PFU, plaque-forming units). Die Ergebnisse wurden als $\log_{10}$ PFU ausgedrückt.

Die Bestimmung der Restaktivität erfolgte, wie in Versuch 1 beschrieben. Die Ergebnisse sind in der Tabelle 7 zusammengefaßt. Sie zeigen, daß der (relativ hitzestabile) Bakteriophage X 174 in einer Gewebeklebstoffpräparation durch trockenes Erhitzen unter $N_2$ auf 90°C vollständig inaktiviert werden kann, wobei die Aktivität der Gewebeklebstoffpräparation nahezu vollständig erhalten bleibt.

# EP 0 183 674 B1

## Tabelle 7

Inaktivierung eines Modellvirus (Bakteriophage X 174) in einer Gewebeklebstoffpräparation mit einem Faktor XIII-Gehalt von 520 Einheiten pro Gramm Fibrinogen und einem Wassergehalt von 0,005 beim Erhitzen unter $N_2$ auf 90°C

| Erhitzung Stunden 90°C | Bakteriophagen X 174- Virustiter ($\log_{10}$ PFU) | Restaktivität | | |
|---|---|---|---|---|
| | | Fibrinogen (Gerinnbares Protein) (mg/ml) | Vernetzung der nach 2 Stunden | Fibrin-$\alpha$-Ketten |
| 0 | 5,8 | 82 | 0,95 | |
| 0,3 | 4,1 | | | |
| 1 | 1,9 | | | |
| 3 | 0 | 83 | 0,90 | |

**Beispiel 5:**

Inaktivierung von Sindbis-Virus und Hundehepatitis-Virus in einer Gewebeklebstoffpräparation durch trockenes Erhitzen auf 110°C

Eine Gewebeklebstoffpräparation wurde, wie in Beispiel 3 beschrieben, hergestellt.

Ein Teil der erhaltenen gefriergetrockneten Gewebeklebstoffpräparation wurde zu einer Proteinkonzentration von 50 mg/ml gelöst, mit einer Sindbis-Virussuspension bzw. einer Hundehepatitis-Virussuspension in Zellkulturmedium TCM 199 bzw. mit virusfreiem Zellkulturmedium versetzt, in Glasfläschchen zu je 2,6 ml abgefüllt, tiefgefroren, bis zu einem Wassergehalt von 0,005 gefriergetrocknet und unter $N_2$ verschlossen. Die Proben wurden bei einer Temperatur von 110°C verschieden lang erhitzt und wie beschrieben, die Aktivität der Präparation und der Virustiter vor dem Erhitzen und zu bestimmten Zeiten während des Erhitzungsvorganges bestimmt.

Die Ergebnisse sind in Tabelle 8 zusammengefaßt.

Das Beispiel zeigt, daß nicht nur Sindbis-Virus, sondern auch das als besonders hitzestabil bekannte Hundehepatitis-Virus in einer Gewebeklebstoffpräparation nach dem erfindungsgemäßen Verfahren vollkommen inaktiviert werden kann, ohne daß zu große Verluste an der biologischen Aktivität des Präparates in Kauf genommen werden müßten.

## Tabelle 8

Inaktivierung von zwei Modellviren (Sindbis-Virus und Hundehepatitis-Virus) in einer Gewebeklebstoffpräparation mit einem Faktor XII-Gehalt von 520 Einheiten pro Gramm Fibrinogen und einem Wassergehalt von 0,005 beim Erhitzen unter $N_2$ auf 110°C

| Erhitzung Minuten 110°C | Virustiter Sindbis | ($\log_{10}$ TCID$_{50}$) Hundehepatitis | Restaktivität | | |
|---|---|---|---|---|---|
| | | | Fibrinogen (Gerinnbares Protein) (mg/ml) | Vernetzung der nach 2 Stunden | Fibrin-$\alpha$-Ketten |
| 0 | 3,6 | 3,4 | 84 | 0,92 | |
| 2 | 2,0 | 1,9 | | | |
| 6 | 1,2 | 1,6 | | | |
| 20 | <1 | <1 | | 0,85 | |
| 60 | <1 | <1 | 83 | 0,78 | |

**Beispiel 6:**

Inaktivierung von drei verschiedenen Modellviren (Sindbis-Virus, VSV und Corona-Virus) in einer Gewebeklebstoffpräparation mit einem Wassergehalt von 0,04 beim Erhitzen unter $N_2$ auf 60°C

Eine Gewebeklebstoffpräparation wurde, wie in Beispiel 3 beschrieben, hergestellt.

Ein Teil der erhaltenen gefriergetrockneten Gewebeklebstoffpräparation wurde zu einer

9

Proteinkonzentration von 50 mg/ml gelöst und je ein Teil der Lösung mit einer Suspension von Sindbis-Virus bzw. einer Suspension von VSV (vesicular stomatitis virus) bzw. einer Suspension von Corona-Virus in Zellkulturmedium TCM 199 bzw. mit virusfreiem Zellkulturmedium versetzt, zu je 2,6 ml in Glasfläschchen abgefüllt, tiefgefroren, gefriergetrocknet, auf einen Wassergehalt von 0,04 eingestellt und unter $N_2$ verschlossen.

Proben der Präparation wurden auf 60°C erhitzt und vor der Erhitzung und nach bestimmten Erhitzungszeiten die Restaktivität und der Virustiter, wie im Beispiel 1 beschrieben, bestimmt.

Die Ergebnisse sind in Tabelle 9 zusammengefaßt.

Unter den angegebenen Bedingungen waren Sindbis-Virus bereits nach einer Erhitzungsdauer von 3 Stunden, VSV und Corona-Virus sogar schon nach 1 Stunde vollständig inaktiviert, wobei die Aktivität der Präparation weitgehend erhalten blieb.

### Tabelle 9

Inaktivierung von drei verschiedenen Modellviren in einer Gewebeklebstoffpräparation mit einem Faktor XIII-Gehalt von 520 Einheiten pro Gramm Fibrinogen und einem Wassergehalt von 0,04 beim Erhitzen unter $N_2$ auf 60°C

| Erhitzung Stunden 60°C | Virus-Titer ($\log_{10}$ TCID$_{50}$) | | | Restaktivität | | |
| | | | | Fibrinogen (Gerinnbares Protein) (mg/ml) | Vernetzung der nach 2 Stunden | Fibrin-α-Ketten |
| | Sindbis | VSV | Corona | | | |
| 0 | 4,1 | 3,9 | 2,8 | 82 | 0,95 | |
| 1 | 3,6 | <1 | 2,1 | | 0,85 | |
| 3 | <1 | <1 | <1 | | 0,82 | |
| 10 | <1 | <1 | <1 | 81 | 0,77 | |

## Patentansprüche

1. Verfahren zur Inaktivierung von vermehrungsfähigen, filtrierbaren Krankheitserregern in Fibrinogen und Faktor XIII enthaltenden Gewebeklebstoffen bei weitgehender Erhaltung ihrer biologischen Aktivität, dadurch gekennzeichnet, daß

Gewebeklebstoffpräparationen, die einen Mindestgehalt von 100 Einheiten Faktor XIII/g Fibrinogen aufweisen, in Gegenwart eines sauerstoffreien inerten Schutzgases, vorzugsweise Stickstoff, oder unter Vakuum in trockenem Zustand bei einer Temperatur im Bereich von 50 bis 120°C während einer Dauer von mindestens 1 min erhitzt werden.

2. Verfahren nach Anspruch 1, dadurch gekennzeichnet, daß der trockene Zustand der Gewebeklebstoffpräparation durch Nachtrocknen mit wasserbindenden Mitteln, wie Phosphorpentoxid, herbeigeführt wird.

3. Verfahren nach Anspruch 1, dadurch gekennzeichnet, daß eine Gewebeklebstoffpräparation durch Fraktionieren von humanem Kryopräzipitat hergestellt und zusätzlich zu dem nativen Gehalt an Faktor XIII weiterer Faktor XIII, vorzugsweise bis zum Erreichen eines Gesamtgehaltes an Faktor XIII von etwa 500 Einheiten/g Fibrinogen, zugegeben wird, wobei die Hitzebehandlung in irgendeiner Stufe des Fraktionierungsprozesses und/oder nach Abfüllung der Präparation in Endbehälter durchgeführt wird.

## Claims

1. Method of inactivating reproductive filterable pathogens in tissue adhesives containing fibrinogen and Factor XIII, with their biologic activity being largely preserved, characterised in that tissue adhesive preparations having a minimum content of 100 units of Factor XIII/g of fibrinogen are heated in the dry state in the presence of an oxygen-free inert protective gas, preferably nitrogen, or under vacuum at a temperature in the range of from 50 to 120°C for a period of time of at least 1 min.

2. Method according to claim 1, characterised in that the dry state of the tissue adhesive preparation is brought about by after-drying with water-binding agents, such as phosphorus pentoxide.

3. Method according to claim 1, characterised in that a tissue adhesive preparation is produced by fractionation of human cryoprecipitate, and that in addition to the native content of Factor XIII further Factor XIII is added, preferably until a total content of Factor XIII of about 500 units/g of fibrinogen is reached, the heat treatment being carried out in any stage of the fractionation process and/or after filling the preparation into final containers.

## Revendications

1. Procédé pour l'inactivation d'agents pathogènes filtrables capables de se propager dans des colles pour tissus contenant du fibrinogène et du facteur XIII en obtenant largement leur activité biologique, caractérisé en ce que des préparations de colles pour tissus, qui présentent une teneur minimale de 100 unités de facteur XIII/g de fibrinogène, sont chauffées pendant une durée d'au moins 1 min à une température dans l'intervalle de 50 à 120°C à l'état sec en présence d'un gaz protecteur inerte exempt d'oxygène, de préférence l'azote, ou sous vide.

2. Procédé selon la revendication 1, caractérisé en ce que l'état sec de la préparation de colle pour tissus est provoqué par séchage final avec des agents fixant l'eau comme le pentoxyde de phosphore.

3. Procédé selon la revendication 1, caractérisé en ce que l'on fabrique une préparation de colle pour tissus par fractionnement de cryo précipité humain et on ajoute encore du facteur XIII en plus de la teneur naturelle en facteur XIII, de préférence jusqu'à atteindre une teneur totale en facteur XIII d'environ 500 unités/g de fibrinogène, le traitement thermique étant mis en oeuvre dans n'importe quelle étape du processus de fractionnement et/ou après transvasement de la préparation dans des récipients finaux.